# EUROPÄISCHE PATENTSCHRIFT

(11) **EP 0 449 271 B1**
(45) Veröffentlichungstag und Bekanntmachung des Hinweises auf die Patenterteilung: **07.06.1995**
(21) Anmeldenummer: 91104914.6
(22) Anmeldetag: 27.03.1991
(51) Int. Cl.: A61F 13/15

(54) **Saugfähiges Wegwerfkleidungsstück**
Absorbent disposable garment
Couche-culotte absorbante jetable

(30) Priorität: 28.03.1990 JP 32326/90
(43) Veröffentlichungstag der Anmeldung: 02.10.1991
(73) Patentinhaber: UNI-CHARM CORPORATION, Kawanoe-shi Ehime-ken (JP)
(72) Erfinder: Nomura, Hironori, Iyomishima-shi, Ehime-ken (JP); Igaue, Takamitsu, Kawaonoe-shi, Ehime-ken (JP); Tanji, Hiroyuki, Kawanoe-shi, Ehime-ken (JP); Sasaki, Tohru, Kawanoe-shi, Ehime-ken (JP)
(74) Vertreter: Sperling, Rüdiger, Dipl.-Ing.

(56) Entgegenhaltungen:
- EP-A- 0 290 945
- EP-A- 0 345 664
- DE-U- 8 704 470
- GB-A- 2 023 067
- US-A- 3 924 626

## Beschreibung

Die Erfindung betrifft ein Wegwerfkleidungsstück mit einer saugfähigen Einlage, die zwischen einer fluiddurchlässigen, dehnbaren oberen Lage und einer fluidundurchlässigen dehnbaren rückwärtigen Lage sandwichartig angeordnet ist, wobei die Einlage in punktförmigen Bereichen, in vorbestimmten Intervallen an der oberen Lage festgelegt ist, jedoch ohne Klebverbindung an der rückwärtigen Lage anliegt, und mit elastischen Teilen im Bereich von Beinöffnungen.

Ein Wegwerfkleidungsstück dieser Art ist aus der US-A-3 924 626 bekannt, worin eine punktweise Befestigung der inneren Lage sowohl an dem Saugkern als auch in den Randbereichen an der äußeren Lage beschrieben ist. Die Paßform dieses Wegwerfkleidungsstücks wird einzig durch das stramme und materialmäßige Anpassen, insbesondere der äußeren flüssigkeitsundurchlässigen Lage bewirkt.

Dieses bekannte Kleidungsstuck hat den Nachteil, daß es sich nicht ausreichend an der Körperform eines Trägers anschmiegen kann, da das Material, insbesondere die äußere flussigkeitsundurchlässige Lage, den Krümmungen der Körperkonturen nicht ohne größere Faltenbildungen folgen kann.

Der Erfindung liegt daher die Aufgabe zugrunde, ein Wegwerfkleidungsstück der genannten Art zu schaffen, das eine gute Paßform des Kleidungsstücks an den Körper des Trägers sicherstellt.

Die Aufgabe wird erfindungsgemäß dadurch gelöst, daß die elastischen Teile für die Beinöffnungen aus ersten und zweiten Elastikteilen bestehen, die sich, bei symmetrischem Aufbau, jeweils fortlaufend mit jeweiligen Teilabschnitten entlang der Beinöffnung erstrecken, sich jeweils auf Höhe der Mitte der Beinöffnung kreuzen und anschließend mit jeweiligen mittleren Teilabschnitten quer über den Schrittbereich verlaufen, wobei die Elastikteile ausschließlich entlang ihrer Teilabschnitte an der oberen und/oder rückwärtigen Lage angeklebt sind und die mittleren Teilabschnitte unbefestigt bleiben.

Durch diese Merkmale ist eine freie Bewegung der rückwärtigen Lage möglich, ohne daß es zu Verspannungen aufgrund von Bewegungen des Trägers kommt, die zu einem Abheben von Teilbereichen des Beinlochs von der Haut führen könnten.

In einem bevorzugten Ausführungsbeispiel umfaßt die vordere Lage ein fluiddurchlässiges dehnbares Faservlies, wohingegen die rückwärtige Lage ein fluiddurchlässiges dehnbares Faservlies und eine fluidundurchlässige, dehnbare Sperrlage, wie beispielsweise einen Kunststoff- oder Gummifilm aufweist und wobei die Einlage bevorzugt eine Mischung aus flockiger Pulpe, supersaugfähigem Polymerpulver und thermoplastischen Fasern besteht, welche Fasern zusammengeschweißt sind und durch die gesamte Mischung in Richtung ihrer Dicke druckgeformt ist.

In einem anderen bevorzugten Ausführungsbeispiel ist dieses Faservlies der Rückseite an der Sperrlage an Punkten, die einen vorbestimmten Abstand voneinander haben, festgeklebt.

Das Merkmal, daß die Einlage nicht an der rückwärtigen Schicht angeklebt ist, ist deshalb vorteilhaft, weil die rückwärtige Lage frei ist, sich ohne Behinderung durch diese Einlage auszudehnen, so daß diese Einlage mit guter Paßform an den Körper des Trägers angepreßt werden kann.

Des weiteren wirkt die Art der Verklebung mit der innerne Funktion der rückseitigen Lage zusammen, um die Einlage wirksam bei gegebenen Umständen an der Bewegung zu hindern.

### KURZE BESCHREIBUNG DER ZEICHNUNGEN

Nachfolgend wird die Erfindung anhand der Zeichnung dargestellter Ausführungsbeispiele näher erläutert. Es zeigen:
- Fig. 1: eine perspektivische Ansicht von Übungshosen,
- Fig. 2: eine perspektivische Ansicht einer Explosionsdarstellung dieser Übungshosen;
- Fig. 3: eine Draufsicht der inneren Oberfläche der oberen Lage; und
- Fig. 4: eine Schnittansicht, die die Saugeinlage bedeckt mit der oberen und der rückwärtigen Lage darstellt.

### BEVORZUGTE AUSFÜHRUNGSFORMEN DER ERFINDUNG

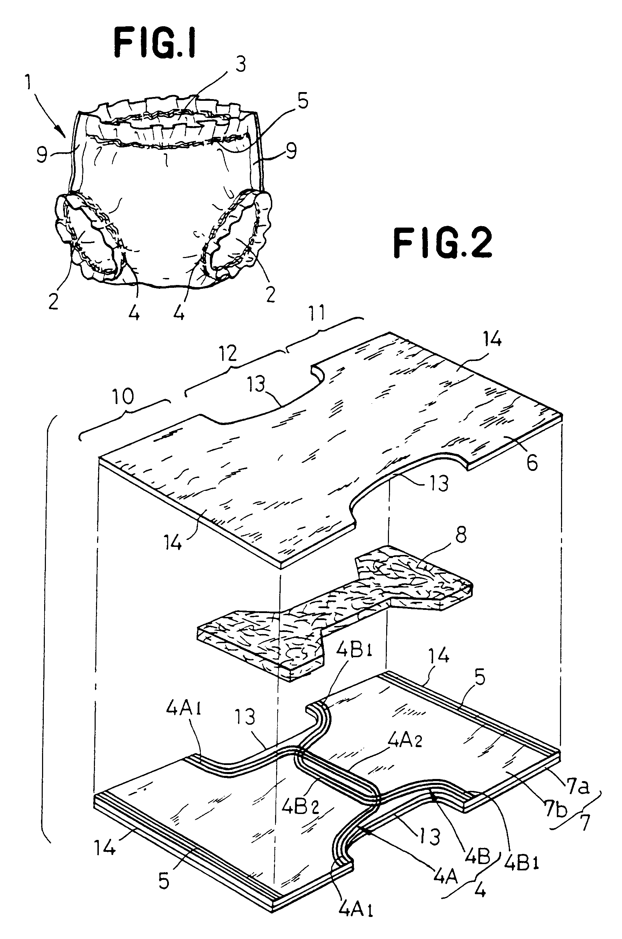

Fig. 1 ist eine perspektivische Ansicht von Übungshosen 1 mit einem Paar Beinöffnungen 2 und einer Hüftöffnung 3, woran jeweils elastische Teile 4,5 angeordnet sind.

Fig. 2 ist eine perspektivische Ansicht einer Explosionsdarstellung der Übungshosen 1 mit einer fluiddurchlässigen dehnbaren oberen Lage 6, einer fluidundurchlässigen dehnbaren rückwärtigen Lage 7, einer saugfähigen Einlage 8 und mit den genannten elastischen Teilen 4,5 für jeweils die Beine und die Hüfte.

Die obere Lage 6 ist ein fluidurchlässiges dehnbares Vlies. Die rückwärtige Lage 7 besteht aus einem fluiddurchlässigen dehnbaren Vlies 7a und einer fluidundurchlässigen dehnbaren Sperrlage, wie beispielsweise einem Kunststoff- oder Gummifilm 7b. Die Einlage 8 besitzt eine sogenannte Sanduhrform besteht aus einer Mischung 2 aus flockiger Pulpe, supersaugfähigem Polymerpulver und thermoplastischen Fasern, wobei diese Fasern miteinander verschweißt und dabei die gesamte Mischung in Richtung der Dicke druckgeformt ist, so daß ihre Beschaffenheit halbsteif ist.

Die einander gegenüberliegenden Ränder eines Schrittbereichs 12 zwischen dem vorderen und dem rückwärtigen Abschnitt, 10,11, der oberen und der rückwärtigen Lage 6,7, sind mit eingekerbten oder vertieften Kanten 13 versehen, um jeweils Beinöffnungen 2 auszubilden. Diese eingekerbten Ränder 13 und ein Hüftrand 14 sind jeweils mit elastischen Teilen 4 und 5 versehen. Das elastische Teil 4 für die Beinöffnungen besteht aus einem ersten Teil 4A und einem zweiten Teil 4B. Die ersten und zweiten Teile 4A,4B schneiden einander an Punkten, die einen Abstand von ihren Enden haben und definieren damit Schnittpunkte neben den Mittelpunkten jeder dieser eingekerbten Ränder 13. Abschnitte 4A₁, 4B₁ der Teile 4A, 4B erstrecken sich von ihren jeweiligen Schnittpunkten zu zugeordneten äußeren Enden hin und sind an der Innenfläche(n) der oberen und/oder rückwärtigen Lage entlang der jeweiligen gekerbten Ränder 13 mit Klebmitteln angeklebt. Mit 4A₂ , 4B₂ gekennzeichnete Abschnitte der Teile 4A, 4B sind nicht an den Komponenten angeklebt, sondern lediglich auf der zentralen Unterseite der Einlage 8 positioniert. Das elastische Teil 5 für die Hüftöffnung ist an der Innenfläche 5 der oberen und/oder rückwärtigen Lage entlang des Hüftrands 14 mit einem heißschmelzenden Adhesiv (nicht dargestellt) angeklebt. Äußere Umfangsränder der oberen und rückwärtigen Lage 6,7 können miteinander durch Klebmittel, wie beispielsweise Klebstoff oder durch Verschweißen (einschl. Ultraschallschweißung) verbunden sein.

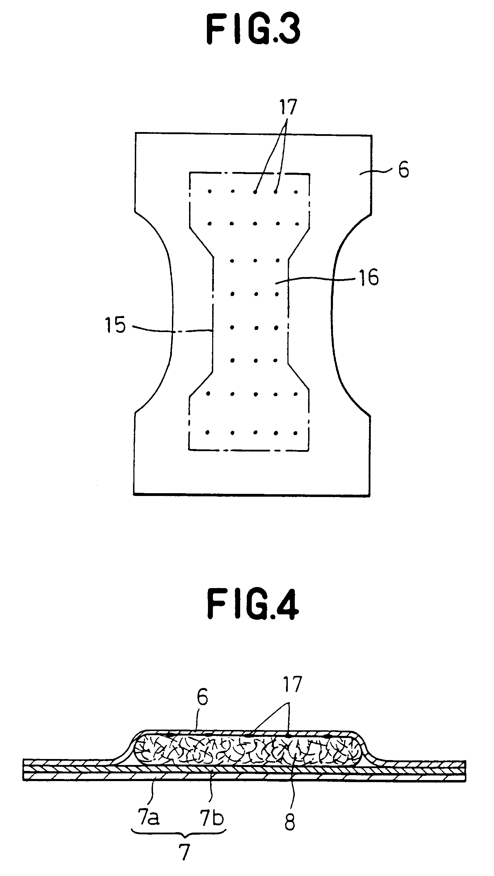

Fig. 3 ist eine Draufsicht auf die Innenfläche der oberen Lage 6. Die Einlage 8 ist in einer Zone 16 positioniert, die mit einer Kettenlinie 15 auf der Innenoberfläche der oberen Lage 6 umgeben ist. Wie in Fig. 4 dargestellt ist, ist die Zone 16 der oberen Lage 6 intermittierend an der Oberfläche der Einlage 8 mittels punktförmiger Klebmittel 17 angeklebt, die in relativ großen Abständen verteilt sind, um zu verhindern, daß die Zone 16 von der Oberfläche der Einlage 8 wegfließt und dabei die innere Übertragung der flüssigen Ausscheidungen durch diese Zone 16 hindurch stört und um zu verhindern, daß die Einlage sich bei Bewegung verschiebt. Die Klebmittel 17 können ebenfalls aus den verschiedenartigen Klebmitteln gewählt werden.

Vorzugsweise ist das Faservlies 7a der rückwärtigen Lage 7 und die Lage 7b intermittierend miteinander an punktförmigen Stellen mittels der Klebmittel verbunden.Falls es gewünscht wird, kann das Vlies 7a in der Zone, die von der Einlage freigelassen ist, inmittierend an der oberen Lage 6 und der Lage 7b intermittierend verklebt sein, mit welcher letzteren Lage das Vlies 7a in unmittelbarem Kontakt steht, und zwar mittels der punktförmigen Klebmittel.

Eine Laminatanordnung, die auf diese Weise als Wegwerfübungshose zusammengestellt ist, kann in Längsrichtung entlang der Mittellinie gefaltet sein, wobei die einander gegenüberliegenden Ränder 9 mit den genannten Klebmitteln verklebt werden, um eine vollständige Übungshose 1, wie in Fig.1 dargestellt, zu bilden. Es versteht sich, daß die vorliegende Erfindung auch als Wegwerfwindel vom offenen Typ ausgelegt sein kann, die Klebebänder aufweist, um die Hüftlinie zu schließen.

## Patentansprüche

1. Wegwerfkleidungsstück mit einer saugfähigen Einlage (8), die zwischen einer fluiddurchlässigen, dehnbaren oberen Lage (6) und einer fluidundurchlässigen dehnbaren rückwärtigen Lage (7) sandwichartig angeordnet ist, wobei die Einlage (8) in punktförmigen Bereichen, in vorbestimmten Intervallen an der oberen Lage festgelegt ist, jedoch ohne Klebverbindung an der rückwärtigen Lage (7) anliegt, und mit elastischen Teilen (4) im Bereich von Beinöffnungen, dadurch **gekennzeichnet**, daß die elastischen Teile (4) für die Beinöffnungen aus ersten (4A) und zweiten (4B) Elastikteilen bestehen, die sich, bei symmetrischem Aufbau, jeweils fortlaufend mit jeweiligen Teilabschnitten (4A₁, 4B₁) entlang der Beinöffnung (13) erstrecken, sich auf Höhe der Mitte der Beinöffnung (13) kreuzen und anschließend mit jeweiligen mittleren Teilabschnitten (4A₂, 4B₂) quer über den Schrittbereich verlaufen, wobei die Elastikteile (4A, 4B) ausschließlich entlang ihrer Teilabschnitte (4A₁, 4B₁) an der oberen und/oder rückwärtigen Lage angeklebt sind und die mittleren Teilabschnitte (4A₂, 4B₂) unbefestigt bleiben.

2. Wegwerfkleidungsstück nach Anspruch 1, wobei die obere Lage (6) ein fluiddurchlässiges, dehnbares Faservlies aufweist, während die rückwärtige Lage ein fluiddurchlässiges, dehnbares Faservlies (7b) und eine fluidundurchlässige dehnbare Sperrlage (7a), wie beispielsweise einen Kunststoff- oder Gummifilm aufweist.

3. Wegwerfkleidungsstück nach Anspruch 2, dadurch gekennzeichnet, daß das Faservlies der rückwärtigen Lage (7) an der Sperrlage (7a) an punktförmigen Stellen in vorbestimmten Abständen festgeklebt ist.

## Claims

1. Disposable garment with an absorbent insert (8) arranged sandwich-like between a fluid-permeable, flexible upper layer (6) and a fluid-impermeable, flexible rear layer (7), the insert (8) being fixed at point-shaped regions to the upper layer at predetermined intervals but abutting the rear layer (7) without any adhesive connection, and the garment having elastic parts (4) in the region of leg openings, characterised in that the elastic parts (4) for the leg openings comprise first (4A) and second (4B) elastic parts which are symmetrically disposed and which each extend with respective portions (4A₁, 4B₁) along the leg opening (13), cross at the middle of the leg opening (13), and then extend with respective middle portions (4A₂, 4B₂) transversely over the crotch region, the elastic parts (4A, 4B) being secured solely along their portions (4A₁, 4B₁) to the upper and/or rear layer and the middle portions (4A₂, 4B₂) remaining free.

2. Disposable garment according to Claim 1, wherein the upper layer (6) is provided with a fluid permeable, flexible non-woven fabric, while the rear layer is provided with a fluid permeable, flexible non-woven fabric (7b) and a fluid impermeable flexible barrier layer (7a), such as for example a plastic or rubber film.

3. Disposable garment according to Claim 2, characterised in that the non-woven fabric of the rear layer (7) is secured to the barrier layer (7a) at point-shaped positions at predetermined intervals.

## Revendications

1. Vêtement jetable, comportant une couche interne absorbante (8) prise en sandwich entre une couche supérieure (6) extensible et perméable aux fluides, et une couche arrière (7) extensible et imperméable aux fluides, ladite couche interne (8) étant fixée à la couche supérieure dans des zones en forme de points et à intervalles prédéterminés, en étant en contact avec la couche arrière (7), mais sans lui être liée par collage, et des éléments élastiques (4) dans la zone des ouvertures de passage des jambes, caractérisé en ce que les éléments élastiques (4) des ouvertures de passage des jambes sont constitués par des éléments élastiques primaires (4A) et secondaires (4B) qui, dans le cas d'un ensemble symétrique, s'étendent de façon continue le long de l'ouverture de passage des jambes (13), avec des portions partielles respectives (4A₁, 4B₁), se croisent au niveau du milieu de l'ouverture de passage des jambes (13), et s'étendent ensuite, avec des portions partielles médianes respectives (4A₂, 4B₂), transversalement et par dessus la zone de l'entrejambe, les éléments élastiques (4A, 4B) étant collés exclusivement le long de leurs portions partielles (4A₁, 4B₁) à la couche supérieure et/ou arrière, et les portions partielles médianes (4A₂, 4B₂) restant non collées.

2. Vêtement jetable selon la revendication 1, dans lequel la couche supérieure (6) présente un non tissé extensible, perméable aux fluides, tandis que la couche arrière comporte un non tissé (7b) extensible, perméable aux fluides, et une couche de barrage (7a) extensible et imperméable aux fluides, comme, par exemple, un film de matière plastique ou de caoutchouc.

3. Vêtement jetable selon la revendication 2, caractérisé en ce que le non tissé de la couche arrière (7) est collé à la couche de barrage (7a) dans des zones en forme de points, à des intervalles prédéterminés.
